# FASCICULE DE BREVET EUROPEEN

(11) **EP 3 008 166 B1**
(45) Date de publication et mention de la délivrance du brevet: **14.02.2018**
(21) Numéro de dépôt: 14729953.1
(22) Date de dépôt: 14.05.2014
(51) Int. Cl.: C12N 1/38, A23K 10/20, A23K 20/147, A23K 50/40, A23K 50/80, A23J 1/04

(54) **LYOPHILISAT DE VER MARIN ET SES UTILISATIONS**
WATTWURM-LYOPHILISAT UND VERWENDUNGEN DAVON
SAND WORM LYOPHILISATE AND USES THEREOF

(30) Priorité: 16.05.2013 FR 1354399
(43) Date de publication de la demande: 20.04.2016
(73) Titulaire: Hemarina, 29600 Morlaix (FR)
(72) Inventeur: ZAL, Franck, F-29600 Ploujean-Morlaix (FR)
(74) Mandataire: Lavoix
(86) Numéro de dépôt international: PCT/FR2014/051118
(87) Numéro de publication internationale: WO 2014/184492

(56) Documents cités:
- WO-A1-2010/128159
- WO-A2-2005/037392
- WO-A2-2006/016135
- DUWAT PATRICK ET AL: "Respiration capacity of the fermenting bacterium Lactococcus lactis and its positive effects on growth and survival", JOURNAL OF BACTERIOLOGY, AMERICAN SOCIETY FOR MICROBIOLOGY, US, vol. 183, no. 15, 1 August 2001 (2001-08-01), pages 4509-4516, XP002462294, ISSN: 0021-9193, DOI: 10.1128/JB.183.15.4509-4516.2001
- BROOIJMANS ROB ET AL: "Heme and menaquinone induced electron transport in lactic acid bacteria", MICROBIAL CELL FACTORIES, BIOMED CENTRAL, GB, vol. 8, no. 1, 29 May 2009 (2009-05-29), page 28, XP021058468, ISSN: 1475-2859, DOI: 10.1186/1475-2859-8-28

## Description

La présente invention a pour objet un lyophilisat ou une poudre comprenant i) au moins une globine, un protomère de globine ou une hémoglobine extracellulaire d'Annélides, et ii) au moins un matériel biologique d'Annélides distinct de ladite globine, dudit protomère et de ladite hémoglobine.

La croissance de micro-organismes à l'échelle industrielle est une préoccupation majeure des technologies qui exploitent ces micro-organismes. En effet, afin de produire des aliments (yaourts, bières, vins), mais aussi des médicaments (pénicilline...) ou des biocarburants (éthanol, biogaz), de nombreux micro-organismes de nature et de propriétés différentes sont utilisés. La culture en bioréacteur nécessite une sélection de la (des) souche(s) de micro-organisme(s) pertinente(s), du substrat, mais aussi des conditions de culture.

L'hémine est souvent utilisée comme additif de culture en bioréacteur. Ce produit est une protoporphyrine IX, ou un analogue chimique ou naturel, contenant du fer et complexée avec du chlore. Il provient de mammifères, notamment de bovins ou de porcins, ou bien est synthétisé par des procédés chimiques, et est notamment utilisé en bioréacteur de fermentations industrielles. Cependant, ce produit est très difficile à solubiliser et à stabiliser, ce qui n'est pas adapté à une culture en bioréacteur. Par ailleurs, ce produit peut être dangereux, notamment du fait de la présence de virus, de bactéries, de levures ou de prions, potentiellement transmissibles ou pathogènes pour l'homme, et n'est absolument pas adapté à la production de produits Casher et/ou Halal par des micro-organismes.

Il existe donc un besoin pour un produit compatible avec la culture en bioréacteur, qui ne soit pas issu de mammifère, et qui soit exempt de pathogène.

Duwat Patrick et al. "Respiration capacity of the fermenting Bacterium Lactococcus lactis and its positive effects on growth and survival", Journal of bacteriology, American society for Microbiology, US, Vol. 183, no. 15, 1 aout 2001 (2001-08-01), pages 4509-4516; et

Brooijmans Rob et al. "Heme and menaquinone induced electron Transport in lactic acid bacteria", microbial cell factories, biomed central, GB, Vol. 8, no. 1, 29 mai 2009 (2009-05-29), page 28, concernent l'utilisation d'hémine ou d'hème afin d'améliorer la viabilité des bactéries lactiques. WO 2010/128159 A1 concerne une hémoglobine isolée de vers appartenant à la famille des Nereididae et son utilisation dans un milieu de culture cellulaire, dans des solutions de conservation et comme transporteur artificiel de l'oxygène pour la transfusion.

La Demanderesse a découvert de façon surprenante que l'utilisation d'un lyophilisat ou d'une poudre, obtenu à partir d'Annélides entiers, ou de coproduits d'Annélides (i.e. produits de vers restants obtenus suite à l'extraction de l'hémoglobine d'Annélides), ou bien encore d'hémoglobine d'Annélides lyophilisée, permet d'obtenir un bon rendement de fermentation, notamment lactique, tout en apportant du fer. Cette molécule ne précipite pas dans le milieu de culture du bioréacteur, et permet donc de maintenir une bonne homogénéité.

Ce lyophilisat présente en outre des propriétés nutritionnelles intéressantes, qui le rendent compatible avec son intégration dans des produits alimentaires, notamment animaux.

La Demanderesse a maintenant trouvé que l'utilisation d'Annélides entiers ou de coproduits d'Annélides, broyés et lyophilisés, permet d'obtenir un bon rendement de fermentation, notamment lactique, tout en apportant du fer. Ces Annélides entiers ou ces coproduits d'Annélides, broyés et lyophilisés, constituent en outre une source d'alimentation animale intéressante.

L'invention se rapporte donc à l'utilisation d'un lyophilisat ou une poudre comprenant i) au moins une globine, un protomère de globine ou une hémoglobine extracellulaire d'Annélides, et ii) au moins un matériel biologique d'Annélides distinct de ladite globine, dudit protomère et de ladite hémoglobine.

De préférence, le lyophilisat ou la poudre est obtenu à partir d'Annélides entiers ou de coproduits d'Annélides, préalablement broyés. Un tel lyophilisat est destiné à être utilisé comme améliorant de la fermentation lactique.

Comme indiqué ci-avant, par « coproduit d'Annélides », on entend la fraction de ver restante après l'extraction de l'hémoglobine dudit Annélide.

Le lyophilisat ou la poudre divulgué dans la présente spécification comprend donc d'une part i) au moins une globine, un protomère de globine ou une hémoglobine extracellulaire d'Annélides.

L'hémoglobine extracellulaire d'Annélides est présente chez les trois classes d'Annélides : les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle.

L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine dont les masses sont comprises entre 15 et 18 kDa et qui sont très similaires aux chaînes de type α et β de vertébrés.

La deuxième catégorie, comptant 36 à 42 éléments, regroupe les chaînes polypeptidiques dites de "structure" ou « linkers » possédant peu ou pas de site actif mais permettant l'assemblage des sous-unités appelées douzièmes ou protomères.

Chaque molécule d'hémoglobine est constituée de deux hexagones superposés que l'on a nommés bicouche hexagonale (hexagonal bilayer) et chaque hexagone est lui-même formé par l'assemblage de six sous-unités (ou "douzièmes" ou « protomères ») en forme de goutte d'eau. La molécule native est formée de douze de ces sous-unités (dodécamère ou protomère). Chaque sous-unité a une masse moléculaire comprise entre 200 et 250 kDa, et constitue l'unité fonctionnelle de la molécule native.

Selon l'invention, le lyophilisat peut également comprendre au moins un protomère de globine de l'hémoglobine extracellulaire d'Annélides. Ledit protomère constitue l'unité fonctionnelle de l'hémoglobine native, comme indiqué ci-dessus.

Enfin, le lyophilisat peut également comprendre au moins une chaîne de globine de l'hémoglobine extracellulaire d'Annélides. Une telle chaîne de globine peut notamment être choisie parmi les chaînes de globine de type Ax et/ou Bx d'hémoglobine extracellulaire d'Annélides.

L'hémoglobine extracellulaire d'Annélides, ses protomères de globine et/ou ses globines peuvent être natifs ou recombinants.

De préférence, la globine, le protomère ou l'hémoglobine extracellulaire d'Annélides est choisie parmi les hémoglobines extracellulaires d'Annélides Polychètes, de préférence parmi les hémoglobines extracellulaires de la famille des *Arenicolidae* et les hémoglobines extracellulaires de la famille des *Nereididae.* Encore plus préférentiellement, les Annélides sont choisis parmi *Arenicola sp* et *Nereis sp,* et plus préférentiellement encore *Arenicola marina* ou *Nereis virens.*

Le lyophilisat ou la poudre divulgué dans la présente spécification comprend également ii) au moins un matériel biologique d'Annélides distinct de ladite globine, dudit protomère et de ladite hémoglobine.

Par « matériel biologique distinct de ladite globine, dudit protomère et de ladite hémoglobine », on entend tout matériel biologique (cellules, tissus ou organes) qui est différent de l'hémoglobine extracellulaire, du protomère et de la globine. Ce matériel biologique provient également d'Annélides, lesdits Annélides pouvant être identiques ou différents de l'Annélide duquel est extrait l'hémoglobine, la globine ou le protomère i). De préférence, ledit matériel biologique ii) est un coproduit d'Annélides.

La présente spécification a également pour objet un procédé de préparation du lyophilisat, obtenu à partir de vers Annélides entiers ou de coproduits d'Annélides. Ce procédé comprend en effet :
i) la congélation d'Annélides entiers ou de coproduits d'Annélides à une température comprise entre -20°C et -100°C pendant un temps d'au moins 24h ;
ii) la sublimation du produit congelé obtenu en i) pendant au moins 2h, sous vide ;
iii) le séchage final du produit sublimé obtenu en ii), jusqu'à l'obtention d'un produit sec ; et
iv) le broyage du produit sec obtenu en iii).

Le cycle de lyophilisation comprend trois étapes :
- la congélation (étape i) du procédé selon l'invention) :
   Cette première phase consiste à congeler la solution de telle façon que l'eau contenue soit transformée en glace.

De préférence, la congélation de l'étape i) du procédé selon l'invention est effectuée à une température comprise entre -20°C et -90°C pendant au moins 24h, de préférence au moins 48h. De préférence, la congélation est effectuée à environ -80°C pendant au moins 24h, de préférence au moins 48h.
- la dessiccation primaire ou sublimation (étape ii) du procédé selon l'invention) :
   L'étape de sublimation permet le passage de la glace présente dans la solution congelée de l'état solide à l'état gazeux, sans étape intermédiaire. La solution congelée est desséchée du fait d'une mise sous vide ; la glace devient alors de la vapeur.

La sublimation se fait à l'aide d'une pompe à vide poussé, d'une pompe mécanique ou d'une cryo-pompe.

De préférence, la sublimation de l'étape ii) est effectuée pendant au moins 4h.
- la dessiccation secondaire ou séchage final (étape iii) du procédé divulgué ici):
   Lorsque la glace est totalement sublimée, la phase de dessiccation secondaire peut débuter. Elle permet d'extraire par désorption les molécules d'eau piégées à la surface des produits séchés.
A la fin de l'étape iii), le lyophilisat obtenu comprend entre 1 et 5% en poids d'eau.
Le broyage de l'étape iii) peut par exemple être réalisé à l'aide d'un mortier, d'un broyeur à hélice ou d'un broyeur à billes pour obtenir une fine poudre.

La poudre divulguée dans la présente spécification peut également être obtenue par tout moyen permettant l'obtention d'une poudre sèche.
Le lyophilisat ou la poudre peuvent être stockés dans des bouteilles ou des flacons de verre ou de plastique, de préférence en verre. Le lyophilisat ou la poudre peut être utilisés comme tels ou mis en solution.
Le lyophilisat ou la poudre selon l'invention est facile à transporter et à stocker, il peut ainsi être facilement reconstitué, et est prêt à être utilisé.

La présente spécification se rapporte également à une solution comprenant le lyophilisat ou la poudre divulgué ici. Une telle solution peut être obtenue par simple mélange du lyophilisat ou de la poudre avec un diluant. Le lyophilisat ou la poudre peut en effet être dilué au moment opportun avec le diluant, afin de restituer le broyat initial. De préférence, le diluant est de l'eau ultrapure. Alternativement, de préférence, le diluant est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.8, de préférence égal à 7,1 ± 0,5, de préférence d'environ 7.35. Ladite solution a de préférence une osmolarité comprise entre 300 et 350, et préférentiellement de 302 mOsmol/L.
Plus préférentiellement, le diluant est choisi parmi l'eau ultrapure et une solution aqueuse comprenant 90 mM de NaCl, 23 Mm de Na-gluconate, 2,5 mM de CaCl₂, 27 mM de Na-acétate, 1,5 mM de MgCl₂, 5 mM de KCl, et un pH de 7,1 ± 0,5, pouvant contenir entre 0 et 100 mM d'antioxydant de type acide ascorbique et/ou gluthation réduit.
Il est également possible de diluer le lyophilisat ou la poudre dans une solution de soude (NaOH), notamment dans une solution de soude de 0,01 à 1 N. La dilution de la poudre ou du lyophilisat selon l'invention dans une solution de soude permet d'une part d'augmenter la solubilité du lyophilisat ou de la poudre, mais également de potentiellement la dépyrogénéiser (i.e. détruire les endotoxines). Le temps de dissolution dépend de la concentration de soude utilisée. Il va généralement de 24 heures pour de faibles concentrations en soude, jusqu'à 1 heure pour de fortes concentrations.

L'invention a pour objet l'utilisation d'un lyophilisat ou d'une poudre selon l'invention, ou d'une solution le comprenant, ou encore d'une hémoglobine d'Annélides lyophilisée ou en poudre, pour améliorer la bioproduction. Par « bioproduction », on entend la production d'un produit biologique à une échelle industrielle, de préférence en bioréacteur. Par « produit biologique », on entend notamment une protéine (ie séquence d'acides aminés comprenant au moins 50 acides aminés. De préférence, la protéine est un anticorps, une hémoglobine ou une enzyme), un peptide (ie séquence d'acides aminés comprenant de 2 à 49 acides aminés), une séquence nucléique ou un plasmide. En effet, le lyophilisat ou la poudre selon l'invention, ou la solution qui le comprend, ainsi que l'hémoglobine, peuvent être utilisés dans les bioréacteurs, pour augmenter le rendement de bioproduction. En outre, le lyophilisat ou la poudre selon l'invention, ou la solution qui le comprend, ainsi que l'hémoglobine, peuvent être ajoutés dans les milieux de culture de cellules, animales ou végétales, par exemple des CHO. Ils permettent notamment d'améliorer la croissance desdites cellules.
L'invention a également pour objet l'utilisation d'un lyophilisat ou d'une poudre selon l'invention, ou d'une solution le comprenant, ou encore d'une hémoglobine d'Annélides lyophilisée ou en poudre, pour améliorer le rendement de la fermentation lactique. La fermentation lactique se fait par des bactéries lactiques, typiquement en bioréacteur. Elle ne nécessite pas d'oxygène (anaérobie), et consiste en la transformation du glucose en acide lactique. Comme démontré en exemple, le lyophilisat selon l'invention, une fois reconstitué, présente une bonne stabilité et une bonne fonctionnalité ; il comprend notamment de l'hémoglobine extracellulaire. Également divulguée ici est l'utilisation d'un lyophilisat ou d'une poudre selon l'invention, ou d'une solution le comprenant, ou encore d'une hémoglobine d'Annélides lyophilisée ou en poudre, comme aliment pour animaux, notamment pour les animaux aquatiques et les animaux de compagnie.
Cet aliment peut se présenter sous forme d'appât, de granules, de pâte ou de croquettes ou de poudre.
Il convient particulièrement comme aliment entier ou comme additif d'aliment pour poissons, ou pour les invertébrés tels que mollusques, crustacés, céphalopodes, coraux, ou encore pour les animaux de compagnie.
L'invention est maintenant illustrée par les exemples suivants, qui ne sont nullement limitatifs.

### Exemple 1 : préparation et dosage de lyophilisats selon l'invention

### Matériel & Méthodes

### Poudres de ver

Les produits testés sont des poudres d'*Arenicola marina* et de *Nereis virens.*

Ces poudres ont été obtenues par lyophilisation et broyage.

Les milieux suivants ont été testés :
- MILIEU 1 : H20 milliQ
- MILIEU 2 : Tampon d'extraction (400 mM NaCl, 2.95 mM KCl, 32 mM MgSO₄ 7H₂0, 11 mM CaCl2, 50 mM Tris base, 10 mM acide ascorbique)
- MILIEU 3 : 1N NaOH (utilisé pour redisperser l'Hémine, hémoglobine d'origine bovine)

### Méthode

1g de poudre a été pesé dans un tube à centrifuger de 15mL. 3mL de solution ont été ajoutés puis homogénéisés, dans un premier temps manuellement puis au vortex. Des photographies ont été prises de ce mélange. Ce mélange a ensuite été centrifugé à 5000g pendant 10 minutes. Le culot et le surnageant ont ensuite été pris en photo. Le surnageant a été récupéré et congelé à -80°C. Etant donné les faibles volumes récupérés, l'expérience a été répétée mais sur 3g de poudre et 9mL de solution dans des tubes à centrifuger de 50mL. Des aliquots ont été congelés à -80°C.

Pour les tests analytiques, du matériel frais a été généré en plus grande quantité. 3g de poudre de ver ont été pesés puis resuspendus dans 15mL de la solution de redispersion. Le matériel a ensuite été centrifugé et le surnageant a été récupéré puis filtré sur 0.8µm et 0.22µm. La fonctionnalité, la pureté et un dosage ont été réalisés sur les échantillons.

### Résultats

### 1. Lyophilisation

Pour 5kg d'Arenicole, 670g de poudre ont été générés soit un facteur de 7.5.

Pour 6kg de Nereis, 921g de poudre ont été générés soit un facteur de 6.5.

### 2. Résultats analytiques

### a) Dosage

Les concentrations en hémoglobine sont comprises entre 3 et 8g/kg de vers frais.

| | Arénicole | | | Nereis | | |
|---|---|---|---|---|---|---|
| | Eau | Tampon Tris | Tampon extraction | Eau | Tampon Tris | Tampon extraction |
| [M101] en g/L avec une dilution au 5ème | 8 | 8 | 11 | 4 | 7 | 9 |
| [M101] en g/kg de poudre de vers | 38 | 40 | 56 | 22 | 36 | 47 |
| [M101] en g/kg de vers frais | 5 | 5 | 8 | 3 | 6 | 7 |

### b) Conductivité

La conductivité de la matière sèche est élevée (∼130mS/cm).

### Exemple 2 : Tests avec les lyophilisats selon l'invention

### Matériel & Méthodes

### Poudres de ver

HEMARINA A: issue de *Arenicola marina*
HEMARINA B: issue de *Nereis virens*

Les poudres ont été obtenues comme dans l'exemple 1.

### Réparation des échantillons

i) Culture en flasks : mise en solution des poudres à 200 g/L puis filtration du surnageant et ajout dans les flasks (i.e. flacons).
ii) Culture en bioréacteurs : ajout direct dans les réacteurs à raison de 1g/L de poudre avant autoclavage.

### Conditions de culture

### i) Culture en flasks (100 mL, 0,5 et 1L) :

La souche utilisée est une souche de Lactococcus lactis SB50.

Les essais ont été réalisés pendant la nuit (16-18h) à 30°C et 180 rpm d'agitation.

La composition du milieu de culture de la souche est la suivante : Glucose (1%), Maltose (0,1%), Extrait de levure (2%) et minéraux.

Les conditions de culture sont les suivantes : remplissage à 10% volume utile ; un témoin positif avec ajout d'hémine (hémoglobine d'origine bovine) à 10 ppm final est utilisé.

Les échantillons HEMARINA (poudres en solution) ont été ajoutés post stérilisation après centrifugation.

### ii) Culture en bioréacteurs (1L vol. utile) :

Les essais ont été réalisés pendant la nuit (15-16h) à 30°C.
La composition du milieu de culture de la souche est identique à celle des flasks, avec un ajout d'antimousse 0,01%.
Les conditions de culture sont les suivantes : remplissage à 60% volume utile, pO2 régulée à 20% ou 80% et pH régulé à 6.
On ajoute directement 1g/L de poudre dans le milieu de culture avant autoclavage.

La densité optique (DO) à 600 nm est mesurée pour chaque culture.

### Résultats

### i) Culture en flasks :

Les DO sont les suivantes :

| **Référence** | **DO à 600 nm** |
|---|---|
| **Témoin (Lc lactis seul)** | 1.52 |
| **Témoin positif (Lc lactis+hémine 10 ppm)** | 4.5 |

La concentration active de chaque produit (HEMARINA A et HEMARINA B) est équivalente à celle de l'hémine, i.e. est d'environ 0.25 ppm.

### ii) Culture en bioréacteurs :

Les résultats sont les suivants :

| | **Témoin** | **Témoin positif** | **HEMARINA A** |
|---|---|---|---|
| **DO à 600 nm** | 10.63 | 10.9 | 12.07 |
| **Poids sec (g/L)** | 2.8 | 3.4 | 4.1 |
| **Volume NH4OH (mL)** | 18.2 | 12.3 | 15.8 |
| **Viabilité (UFC/mL)** | 1.75E+09 | 5.03E+09 | 6.42E+09 |

La viabilité de la souche est améliorée de 3 à 4 fois en présence des produits HEMARINA A ou HEMARINA B.

La production d'acide lactique (correspondant au volume de NH4OH produit) est inférieure au témoin mais supérieure au témoin positif (hémine).

Le pouvoir acidifiant des produits HEMARINA A et HEMARINA B est équivalent à celui du témoin positif.

**En conclusion, il apparaît que les produits HEMARINA A et HEMARINA B permettent d'améliorer la viabilité de souches lactiques, et ont un très bon pouvoir acidifiant.**

**Ces produits pourraient se substituer aux hémines bovines et porcines, qui sontlargement utilisées dans les domaines agroalimentaires mais qui présentent des risques potentiels de contamination virale ou de type prion à l'homme, et qui ne sont pas adaptées à la production de produits Casher et/ou Halal.**

## Revendications

1. Utilisation d'un lyophilisat ou d'une poudre comprenant i) au moins une globine, un protomère de globine ou une hémoglobine extracellulaire d'Annélides, et ii) au moins un matériel biologique d'Annélides distinct de ladite globine, dudit protomère et de ladite hémoglobine, ou d'une solution comprenant ledit lyophilisat ou ladite poudre, ou encore d'une hémoglobine d'Annélides lyophilisée ou en poudre, pour améliorer le rendement de la fermentation lactique par des bactéries lactiques.

2. Utilisation d'un lyophilisat ou d'une poudre comprenant i) au moins une globine, un protomère de globine ou une hémoglobine extracellulaire d'Annélides, et ii) au moins un matériel biologique d'Annélides distinct de ladite globine, dudit protomère et de ladite hémoglobine, ou d'une solution comprenant ledit lyophilisat ou ladite poudre, ou encore d'une hémoglobine d'Annélides lyophilisée ou en poudre, pour améliorer le rendement de bioproduction de protéines en bioréacteur.

3. Utilisation selon la revendication 1 ou 2, dans lequel l'Annélide est choisi parmi les Annélides Polychètes, de préférence parmi la famille des *Arenicolidae* et des *Nereididae,* plus préférentiellement parmi *Arenicola marina* et *Nereis virens.*

4. Utilisation selon l'une des revendications 1 à 4, dans laquelle le lyophilisat est obtenu par un procédé comprenant :
i) la congélation d'Annélides entiers ou de coproduits d'Annélides à une température comprise entre -20°C et -100°C pendant un temps d'au moins 24h ;
ii) la sublimation du produit congelé obtenu en i) pendant au moins 2h, sous vide ;
iii) le séchage final du produit sublimé obtenu en ii), jusqu'à l'obtention d'un produit sec ; et
iv) le broyage du produit sec obtenu en iii).

5. Utilisation selon la revendication 4, dans laquelle la congélation de l'étape i) est effectuée à une température comprise entre -20°C et -90°C pendant au moins 24h.

6. Utilisation selon la revendication 4 ou 5, dans lequel la sublimation de l'étape ii) est effectuée pendant au moins 4h.

## Patentansprüche

1. Verwendung eines Lyophilisates oder eines Pulvers, umfassend i) mindestens ein Globin, ein Protomer von Globin oder ein extrazelluläres Hämoglobin von Anneliden, und ii) mindestens ein biologisches Material von Anneliden, das sich unterscheidet von dem besagten Globin, dem besagten Protomer und dem besagten Hämoglobin, oder von einer Lösung, die das besagte Lyophilisat oder das besagte Pulver enthält, oder auch von einem lyophilisierten oder pulverisierten Hämoglobin von Anneliden, um die Ausbeute der Milchsäurefermentation durch Milchsäurebakterien zu verbessern.

2. Verwendung eines Lyophilisates oder eines Pulvers, umfassend i) mindestens ein Globin, ein Protomer von Globin oder ein extrazelluläres Hämoglobin von Anneliden, und ii) mindestens ein biologisches Material von Anneliden, das sich unterscheidet von dem besagten Globin, dem besagten Protomer und dem besagten Hämoglobin, oder von einer Lösung, die das besagte Lyophilisat oder das besagte Pulver enthält, oder auch von einem lyophilisierten oder pulverisierten Hämoglobin von Anneliden, um die Ausbeute von Bioproduktion von Proteinen im Bioreaktor zu verbessern.

3. Verwendung nach Anspruch 1 oder 2, in welcher die Annelida unter den Vielborstigen Anneliden ausgewählt werden, vorzugsweise aus der Familie der *Arenicolidae* und der *Nereididae,* noch bevorzugter unter *Arenicola marina* und *Nereis virens.*

4. Verwendung nach einem der Ansprüche 1 bis 4, in welcher das Lyophilisat durch ein Verfahren gewonnen wird, das umfasst:
i) die Gefrierung von ganzen Anneliden oder von Nebenprodukten von Anneliden bei einer Temperatur zwischen -20 °C und -100 °C während einer Zeit von mindestens 24 h;
ii) die Sublimation des unter i) erhaltenen gefrorenen Produkts während mindestens 2 h unter Vakuum;
iii) die abschließende Trocknung des unter ii) erhaltenen sublimierten Produkts, solange bis ein Trockenprodukt erhalten wird; und
iv) die Zerkleinerung des unter iii) erhaltenen Trockenprodukts.

5. Verwendung nach Anspruch 4, in welcher die Gefrierung des Schrittes i) bei einer Temperatur zwischen -20 °C und -90 °C während mindestens 24 h durchgeführt wird.

6. Verwendung nach Anspruch 4 oder 5, in welcher die Sublimation des Schrittes ii) während mindestens 4 h durchgeführt wird.

## Claims

1. The use of a lyophilisate or of a powder comprising i) at least one globin, one globin protomer or one extracellular hemoglobin from Annelids, and ii) at least one biological material from Annelids that is different from said globin, from said protomer and from said hemoglobin, or of a solution comprising said lyophilisate or said powder, or else of a lyophilized or powdered hemoglobin from Annelids, for improving the yield of lactic fermentation by lactic acid bacteria.

2. The use of a lyophilisate or of a powder comprising i) at least one globin, one globin protomer or one extracellular hemoglobin from Annelids, and ii) at least one biological material from Annelids that is different from said globin, from said protomer and from said hemoglobin, or of a solution comprising said lyophilisate or said powder, or else of a lyophilized or powdered hemoglobin from Annelids, for improving the bioproduction yield of proteins in bioreactor.

3. The use as claimed in claim 1 or 2, wherein the Annelid is chosen from Polychaete Annelids, preferably from the family *Arenicolidae* and *Nereididae,* more preferentially from *Arenicola marina* and *Nereis virens.*

4. The use as claimed in any one of claims 1 to 4, wherein the lyophilizate is obtained by a process comprising:
i) the freezing of whole Annelids or of Annelid coproducts at a temperature of between -20°C and -100°C for a time of at least 24h;
ii) the sublimation of the frozen product obtained in i) for at least 2h, under vacuum;
iii) the final drying of the sublimated product obtained in ii), until a dry product is obtained; and
iv) the milling of the dry product obtained in iii).

5. The use as claimed in claim 4, wherein the freezing in step i) is carried out at a temperature of between -20°C and -90°C for at least 24h.

6. The process as claimed in claim 4 or 5, wherein the sublimation in step ii) is carried out for at least 4h.
